# EUROPEAN PATENT APPLICATION

(11) **EP 4 105 239 A1**
(43) Date of publication of application: **21.12.2022**
(21) Application number: 21783819.2
(22) Date of filing: 06.04.2021
(51) Int. Cl.: C07K 16/44, C12N 15/31, G01N 33/53, G01N 33/543, G01N 33/569

(54) **MYCOPLASMA PNEUMONIAE IMMUNOASSAY METHOD AND IMMUNOASSAY INSTRUMENT**

(30) Priority: 08.04.2020 JP 2020069825
(71) Applicant: Denka Company Limited, Tokyo 103-8338 (JP)
(72) Inventor: KOHIYAMA, Risa, Gosen-shi, Niigata 959-1834 (JP); TAKANO, Tomomi, Gosen-shi, Niigata 959-1834 (JP); MIYAZAWA, Takashi, Gosen-shi, Niigata 959-1834 (JP)
(74) Representative: Plougmann Vingtoft a/s
(86) International application number: PCT/JP2021/014591
(87) International publication number: WO 2021/206079

(57) **Abstract**

An immunoassay utilizing a monoclonal antibody which specifically reacts with both of P1 protein and P30 protein of *Mycoplasma pneumoniae,* which monoclonal antibody has a higher affinity with P1 protein and P30 protein of *Mycoplasma pneumoniae* than the known monoclonal antibodies, is disclosed. The immunoassay utilizes an antigen-antibody reaction between a monoclonal antibody which specifically reacts with P1 protein and P30 protein of *Mycoplasma pneumoniae* and which specifically reacts with a peptide containing an amino acid sequence composed of PPQPG or antigen-binding fragment thereof, and P1 protein and P30 protein derived from *Mycoplasma pneumoniae.*

## Description

### TECHNICAL FIELD

The present invention relates to an immunoassay of *Mycoplasma pneumoniae,* and an immunoassay instrument and a monoclonal antibody therefor.

### BACKGROUND ART

Since monoclonal antibodies recognize only a specific antigen, they are widely used for the detection of the specific antigen. For example, an immunoassay of *Mycoplasma pneumoniae,* which immunoassay employs a sandwich method in which a monoclonal antibody that recognizes P30 protein of *Mycoplasma pneumoniae* is immobilized on a solid phase, is described in Patent Document 1.

A polyclonal antibody is a mixture of various antibodies which recognize various antigens or antigenic determinant regions, while a monoclonal antibody recognizes only a certain region of a certain antigen. Because of this property, a monoclonal antibody generally exhibits a higher specificity than a polyclonal antibody. On the other hand, it is easily assumed that a monoclonal antibody is inferior in the kinds and total number of antigens which can bind to the antibody as compared to a polyclonal antibody. This results in low sensitivity of a monoclonal antibody as well as an immunoassay and an immunoassay instrument using it.

To overcome this problem, the present applicant invented a monoclonal antibody which specifically reacts with both of P1 protein and P30 protein of *Mycoplasma pneumoniae*, and filed a patent application (Patent Document 2).

### PRIOR ART REFERENCES

### PATENT DOCUMENTS

Patent Document 1: WO 2015/025968
Patent Document 2: WO 2016/194797

### SUMMARY OF THE INVENTION

### PROBLEMS TO BE SOLVED BY THE INVENTION

Although the monoclonal antibody described in Patent Document 2 is a useful monoclonal antibody which specifically reacts with both of P1 protein and P30 protein of *Mycoplasma pneumoniae,* needless to say, it is advantageous if a monoclonal antibody which has a higher affinity with P1 protein and P30 protein exists because the sensitivity of the immunoassay can further be promoted.

An object of the present invention is to provide a novel monoclonal antibody which specifically reacts with both of P1 protein and P30 protein of *Mycoplasma pneumoniae,* which has a higher affinity with P1 protein and P30 protein of *Mycoplasma pneumoniae* than the monoclonal antibody described in Patent Document 2, and to provide an immunoassay instrument therefor.

### MEANS FOR SOLVING THE PROBLEMS

The present inventors intensively studied to discover that *Mycoplasma pneumoniae* can be assayed with a higher sensitivity than the immunoassay described in Patent Document 2 by using a monoclonal antibody which specifically reacts with P1 protein and P30 protein of *Mycoplasma pneumoniae* and which specifically reacts with a peptide containing an amino acid sequence composed of PPQPG, thereby completing the present invention.

That is, the present invention provides the following:
(1) An immunoassay method of *Mycoplasma pneumoniae,* which utilizes an antigen-antibody reaction between a monoclonal antibody that specifically reacts with P1 protein and P30 protein of *Mycoplasma pneumoniae* and that specifically reacts with a peptide containing an amino acid sequence composed of PPQPG, or an antigen-binding fragment thereof, and P1 protein and P30 protein derived from *Mycoplasma pneumoniae.*
(2) The immunoassay method of (1), wherein said amino acid sequence of said peptide is PPQPGFPPKR or GMPPQPGFPPKR.
(3) The method of (1), wherein said immunoassay method is a sandwich method in which said monoclonal antibody or an antigen-binding fragment thereof is used in at least one of a label and a solid phase.
(4) The method of (2), wherein said immunoassay method is an immunochromatography method.
(5) The method of any one of claims (1) to (4), wherein said two or more kinds of antigens recognized by said monoclonal antibody form a composite.
(6) An immunoassay instrument for carrying out the method of (1), wherein said monoclonal antibody or an antigen-binding fragment thereof is used in at least one of a label and a solid phase.
(7) The immunoassay instrument of (6), wherein said immunoassay instrument is an immunochromatographic strip.
(8) The immunoassay instrument of (6) or (7), wherein said test subject is *Mycoplasma pneumoniae.*
(9) A monoclonal antibody which specifically reacts with P1 protein and P30 protein of *Mycoplasma pneumoniae* and which specifically reacts with a peptide containing an amino acid sequence composed of PPQPG.

### EFFECT OF THE INVENTION

The method of the present invention has an improved detection sensitivity when compared with the method of Patent Document 2. By the present invention, an immunoassay instrument and a monoclonal antibody used for the novel detection method of the present invention are also provided.

### MODE FOR CARRYING OUT THE INVENTION

The test subject detected by the method of the present invention is *Mycoplasma pneumoniae,* and antigens are P1 protein and P30 protein. It should be noted that even if the test subject is quantified or semiquantified, quantification and semiquantification are included in "detection" as used in the present invention because they inevitably accompany "detection".

In the method of the present invention, an immunoassay is performed using a monoclonal antibody or an antigen-binding fragment thereof which specifically reacts with the above-mentioned two kinds of antigens. The term "specifically react" herein means to undergo antigen-antibody reaction, and that in a liquid system in which a protein and an antibody thereof are mixed together, the antibody does not cause an antigen-antibody reaction with the protein component which is an antigen at a detectable level, or merely causes obviously weaker reaction than the antigen-antibody reaction of the antibody with the corresponding antigen thereof even if it causes some binding reaction or association reaction.

An antigen-binding fragment obtained by separating only the antigen binding site from the monoclonal antibody of the present invention can be used in the method of the present invention. That is, cases where a fragment having a specific antigen-binding property (an antigen-binding fragment) such as a Fab, a Fab', a F(ab')₂ or a single-chain antibody (scFᵥ) produced by a known method is used are within the scope of the present invention. Further, the class of the monoclonal antibody is not limited to IgG, and may be IgM or IgY.

The monoclonal antibody used in the method of the present invention can be obtained by immunizing an animal to be immunized with a composite or extract having the above-described two kinds of antigens, or one antigen of the two kinds of antigens or a partial peptide thereof by a known immunological technique and preparing a hybridoma using cells of the immunized animal. Although the length of the peptide used in the immunization is not particularly limited, a peptide having preferably 5 or more amino acids, more preferably 10 or more amino acids can be used as an immunogen. The immunogen can be obtained from a culture fluid, or obtained by incorporating a DNA encoding an arbitrary antigen into a plasmid vector and introducing it into a host cell to express. An arbitrary antigen or a partial peptide thereof used as an immunogen can be expressed as a fusion protein with a protein exemplified below and used as an immunogen after or without purification. Glutathione S-transferase (GST), maltose binding protein (MBP), thioredoxin (TRX), Nus tag, S tag, HSV tag, FRAG tag, polyhistidine tag and the like, which are commonly used as "protein expression/purification tags" by those skilled in the art can be used in the preparation of the fusion protein. Preferably, the fusion protein with the tag is used as an immunogen after it is cleaved to the arbitrary antigen or a partial peptide thereof and the other tag portion by a digestive enzyme, and after separation and purification.

The preparation of the monoclonal antibody from the immunized animal can be easily performed by the well-known method of Kohller et al. (Kohler et al., Nature, vol, 256, p495-497(1975)). That is, antibody producing cells such as splenocytes and lymphocytes are collected from the immunized animal and fused with mouse myeloma cells to produce hybridomas in the usual manner. The resulting hybridomas are cloned by a limiting dilution method or the like. Then, monoclonal antibodies that undergo antigen-antibody reaction with the antigen used in immunizing the animal are selected from the monoclonal antibodies produced from each cloned hybridoma.

Since the monoclonal antibody used in the method of the present invention recognizes two kinds of antigens, the monoclonal antibodies selected in this way, which recognize one of the two kinds of antigens, are screened for a monoclonal antibody that recognizes the other antigen. The monoclonal antibody used in the method of the present invention can be obtained by screening a monoclonal antibody which recognizes both of the two antigens, and which specifically reacts with a peptide containing an amino acid sequence composed of PPQPG (SEQ ID NO: 1). The peptide used in the screening is a peptide containing an amino acid sequence composed of PPQPG, for example, a peptide composed of an amino acid sequence PPQPGFPPKR (SEQ ID NO:2) or GMPPQPGFPPKR (SEQ ID NO:3).

For the purification of a monoclonal antibody from an ascites or a culture supernatant, a known immunoglobulin purification method can be used. Examples of the method include fractionation methods by salting out using ammonium sulfate or sodium sulfate, PEG fractionation methods, ethanol fractionation methods, DEAE ion exchange chromatography methods and gel filtration methods. It is also possible to perform the purification by an affinity chromatography method using a carrier to which any one of Protein A, Protein G and Protein L is bound depending on the species of the immunized animal and the class of the monoclonal antibody.

In the immunoassay of the present invention, the assay is performed by an immunoassay utilizing the antigen-antibody reaction of the monoclonal antibody or the antigen-binding fragment thereof which specifically reacts with the two kinds of antigens prepared as described above (hereinafter, in the descriptions before the examples, "antibody" refers to "antibody or antigen-binding fragment thereof' unless the context clearly dictates otherwise) with *Mycoplasma pneumoniae* in the sample. Any method well known by those skilled in the art such as a competitive method, an agglutination method, a western blotting method, an immunostaining method or a sandwich method can be used as the immunoassay therefor. In the present invention, "assay" includes all of quantification, semiquantification and detection.

As the immunoassay, the sandwich method is preferable. The sandwich method itself is well known in the field of immunoassay and can be performed by an immunochromatography or ELISA. Each of these sandwich methods is well known and the method of the present invention can be performed according to a well-known sandwich method except that the monoclonal antibody of the present invention as described above, which recognizes two or more kinds of antigens, is used.

In a sandwich method, two kinds of antibodies that recognize antigens (an antibody immobilized on a solid phase and a labeled antibody) are used, and in the method of the present invention, at least one of the two kinds of antibodies is the monoclonal antibody which recognizes the two kinds of antigens as described above. As described below, the monoclonal antibody which recognizes the two kinds of antigens as described above is preferably used at least as the immobilized antibody in order to better accomplish the sensitivity improvement that is an object of the present invention, because the antibody immobilized on a solid phase is limited in the amount of the antibody which can be immobilized per area. In cases where at least two kinds of antigens recognized by the monoclonal antibody are present in a single molecule or a single composite, the sandwich method can be performed using a single kind of the monoclonal antibody as the immobilized antibody and the labeled antibody.

In the immunoassay whose detection principle is the sandwich method, anything on which an antibody can be immobilized by a known method can be used as the solid phase on which the antibody is immobilized. For example, known one such as a porous thin film (membrane) with capillary action, a particulate matter, a test tube and a resin flat plate can be arbitrarily selected. As a substance labeling the antibody, an enzyme, a radioactive isotope, a fluorescent substance, a luminescent substance, a colored particle, a colloidal particle or the like can be used. Among the immunoassays using the above-mentioned various materials, a lateral flow immunoassay using a membrane is particularly preferable in view of simplicity and rapidness of clinical examination.

The present invention also provides an immunoassay instrument that enables the immunoassay to be performed in lateral flow manner using the monoclonal antibody which recognizes two or more kinds of antigens. The immunoassay instrument provided by the present invention comprises a support having a detection region in which an antibody (antibody 1) that captures a target to be measured (antigen) is immobilized, a labeled substance region having a movable labeled antibody (antibody 2), a sample pad to which a sample is added dropwise, an absorption band which absorbs the developed sample liquid, and a backing sheet for attaching these members together, wherein at least one of the antibody 1 and the antibody 2 is the monoclonal antibody of the present invention which recognizes two or more kinds of antigens.

The number of the detection regions and the type of the labeled antibodies included in the labeled substance region are not limited to 1. By using antibodies corresponding to a plurality of targets to be measured, two or more kinds of antigens can be detected by the same immunoassay instrument.

The principle of improving the sensitivity of the immunoassay by the method of the present invention is considered as follows. In a sandwich method, an antibody immobilized on a solid phase is used, but the amount of the antibody that can be immobilized per unit area of the solid phase is limited. Also, the time of antigen-antibody reaction is limited. In particular, in an immunochromatography method, since the antigen-antibody reaction occurs only while a specimen or a sample prepared using a specimen (a dilution of a specimen or the like) flows from the upstream and passes through the detection regions, an antigen which cannot be bound to the antibody within this time flows to the downstream of the detection regions as it is and is not detected. When the amount of an antigen in the sample is small, the use of a monoclonal antibody that recognizes two or more kinds of antigens as an immobilized antibody improves the sensitivity more than the use of one kind of monoclonal antibody that recognizes one kind of antigen as the immobilized antibody (ordinary sandwich method), because the amount of the antigen bound to the antibody is increased. In addition, when two kinds of monoclonal antibodies each recognizing one kind of antigen are used in combination as the immobilized antibodies, the amount of each monoclonal antibody immobilized is each half of the total amount of the immobilized antibodies. Therefore, when the time of the antigen-antibody reaction is short, the probability that an antigen collides with and binds to a monoclonal antibody capable of binding to this antigen in a prescribed direction is 1/2 as compared with a case where the entire immobilized antibody is the monoclonal antibody that recognizes the antigen. On the other hand, when a monoclonal antibody that recognizes two or more kinds of antigens is used, any of two or more kinds of antigens will bind to the antibody if it collides with the immobilized antibody in a prescribed direction. Therefore, the amount of the antigens captured by the immobilized antibody is larger than that in cases where two kinds of monoclonal antibodies each recognizing one kind of antigen are used in combination, and thus the sensitivity of the immunoassay is improved.

### EXAMPLES

Hereinafter, the present invention will be described more specifically by way of examples thereof. However, the present invention is not limited to the following examples.

### <Example 1> Preparation of Anti-Mycoplasmapneumoniae Monoclonal Antibody 1. Preparation of Mycoplasma pneumoniae Antigen

*Mycoplasma pneumoniae* was cultured and the culture fluid inactivated by heat treatment at 60°C for 30 minutes was used as an antigen.

### 2. Preparation of Anti-Mycoplasma pneumoniae Monoclonal Antibody

A BALB/c mouse was immunized with the mycoplasma inactivated antigen as described in section 1 above, and the spleen was excised from the mouse bred for a certain period of time and fused with mouse myeloma cells (P3 × 63) by the method of Kohler et al. (Kohler et al., Nature, vol 256, p495-497 (1975)). The obtained fused cells (hybridomas) were maintained in an incubator at 37°C, and cell purification (monocloning) was performed while confirming the antibody activity of the supernatant by ELISA using a plate in which a *Mycoplasma pneumoniae* P1 antigen was immobilized and a plate in which a *Mycoplasma pneumoniae* P30 antigen was immobilized.

As a result, as shown in Table 1, a plurality of hybridoma cell lines producing anti-*Mycoplasma pneumoniae* P1 antibodies, anti-*Mycoplasma pneumoniae* P30 antibodies and anti-*Mycoplasma pneumoniae* P1-P30 antibodies were obtained.

**[Table 1]**

| Clone Name | Reacting Antigen |
|---|---|
| P1-30Aab | P1,P30 |
| P1-30Bab | P1,P30 |
| Plab | P1 |
| P30ab | P30 |

P1 and P30 used in this ELISA were prepared by gel filtration and ion exchange chromatography. The obtained cell lines were intraperitoneally administered to a pristane-treated BALB/c mouse, and about 2 weeks later, antibody-containing ascitic fluid was collected. IgG was purified from the obtained ascitic fluid by an affinity chromatography using a protein A column, and a plurality of purified anti-*Mycoplasma pneumoniae* monoclonal antibodies were obtained.

### <Example 2>

### [Epitope Analysis by ELISA using Synthetic Peptides]

The following peptides were prepared from the amino acid repeating sequence site of P30.

### (Synthetic Peptides)

| | |
|---|---|
| Peptide 1-1: | GFPPQPGMAP (SEQ ID NO:4) |
| Peptide 1-2: | QPGMAPRPGM (SEQ ID NO:5) |
| Peptide 1-3: | APRPGMPPHP (SEQ ID NO:6) |
| Peptide 1-4: | GMPPHPGMAP (SEQ ID NO:7) |
| Peptide 1-5: | HPGMAPRPGF (SEQ ID NO:8) |
| Peptide 1-6: | APRPGFPPQP (SEQ ID NO:9) |
| Peptide 1-7: | APRPGMQPPR (SEQ ID NO:10) |
| Peptide 1-8: | GMQPPRPGMP (SEQ ID NO:11) |
| Peptide 1-9: | PGMPPQPGFP (SEQ ID NO:12) |
| Peptide 1-10: | PPQPGFPPKR (SEQ ID NO:2) |
| Peptide 1-11: | PGMAPRPGMPPH (SEQ ID NO:13) |
| Peptide 1-12: | PGMAPRPGFPPQ (SEQ ID NO:14) |
| Peptide 1-13: | GMPPQPGFPPKR (SEQ ID NO:3) |

The monoclonal antibodies P1-30Aab and P1-30Bab obtained in Example 1 above were evaluated for the reaction with the above-described synthetic peptides by ELISA described below. For the measurement by ELISA, a 96-well plate (NUNC) on which the synthetic peptides were immobilized was used. Each synthetic peptide (2.0 µg/mL) was added to the plate at 100 µl/well, and allowed to react overnight. Thereafter, the plate was washed with 1 × TBS buffer, and Blocking Buffer (1% BSA/TBS) was added at 200 µL/well. Blocking Buffer was discarded and the antibody which was a dilution of that prepared in Example 1 2 was added at 100 µL/well, and allowed to react for 30 minutes. Thereafter, the plate was washed with 200 µL of 1 × washing buffer, and a labeled antibody solution which was HRP-labeled Polyclonal Rabbit Anti-Mouse Immunoglobulins (P0260; Dako) was added at 100 µL/well. Then TBS (pH 7.0) containing 2 w/v% BSA was added at 50 µL/well, and the resultant was allowed to react for 30 minutes. Then the plate was washed with Buffer II, and a substrate solution containing OPD which is a substrate of HRP was added at 100 µL/well, followed by leaving the plate to stand for 10 minutes and addition of 2N sulfuric acid at 100 µL/well to stop the reaction. The absorbance at 450 nm to 630 nm of the reaction solution in each well was measured using a microplate reader.

As a result, any of the antibodies showed reactivity with the amino acid repeat sequence site of P30. Monoclonal antibody P1-30Aab (a monoclonal antibody of the present invention) strongly reacted with peptide 1-10 and peptide 1-13. It also reacted with peptide 1-1 and 1-9. From these results, it was confirmed that monoclonal antibody P1-30Aab has a reactivity with the peptide sequences containing "PPQPG (Pro-Pro-Gln-Pro-Gly)". On the other hand, although monoclonal antibody P1-30Bab showed reactivity with P1, PPQPG does not exist in the amino acid sequence of P1. However, instead, PPHP exists as a similar sequence.

### <Example 3> Immunoassay Instrument for Measuring Mycoplasma pneumoniae 1. Immobilization of Anti-Mycoplasma pneumoniae Antibody on Nitrocellulose Membrane

A solution in which the antibody prepared in Example 1 was diluted to a concentration of 1.0 mg/mL with purified water, and an anti-mouse IgG antibody were provided. The antibody and the anti-mouse IgG antibody were applied linearly on the sample pad side and the absorption body side of a nitrocellulose membrane lined with a PET film, respectively. Thereafter, the nitrocellulose membrane was dried at 45°C for 30 minutes to obtain an anti-*Mycoplasma pneumoniae* antibody-immobilized membrane. In this example, it is referred to as an antibody-immobilized membrane.

### 2. Immobilization of Anti-Mycoplasma pneumoniae Antibody on Colored Polystyrene Particles

The antibody prepared in Example 1 was diluted with purified water to a concentration of 1.0 mg/mL, and colored polystyrene particles were added thereto to a concentration of 0.1%. After stirring, carbodiimide was added to a concentration of 1%, and the resultant was further stirred. The supernatant was removed by centrifugation and the precipitate was resuspended in 50 mM Tris (pH 9.0), 3% BSA to obtain an anti-*Mycoplasma pneumoniae* antibody-bound colored polystyrene particles. In this example, it is referred to as an antibody-immobilized particles.

### 3. Preparation of Test Strips for Measuring Mycoplasma pneumoniae

The antibody-immobilized membranes prepared in section 1 were attached to another members (the backing sheet, the absorption body, the sample pad) and cut to a width of 5 mm to prepare *Mycoplasma pneumoniae* test strips. These are referred to as test strips in this example.

### Sensitivity of Immunochromatography

### <Example 4> Comparison of Sensitivities of Immunoassay Instruments for Measuring Mycoplasma pneumoniae

Three types of test strips, that is, P1P30 test strip, P30 test strip and P1 × P1P30 test strips, were prepared by combining the anti-P1-P30 monoclonal antibody (P1-30Aab, P1-30Bab), anti-P1 monoclonal antibody (Plab) and anti-P30 monoclonal antibody (P30ab) as shown in Table 2, which antibodies were obtained in Example 1.

**[Table 2]**

| | Antibody Used | |
|---|---|---|
| | Antibody-immobilized Membrane | Antibody-immobilized Particles |
| P1 | P1ab | P1ab |
| P30 | P30ab | P30ab |
| P1P30A | P1-30Aab | P1-30Aab |
| P1P30B | P1-30Bab | P1-30Bab |

To each test strip, 50 µL of a sample suspension containing an arbitrarily diluted *Mycoplasma pneumoniae* antigen and the antibody-immobilized particles prepared in section 2 was added dropwise, and the resultant was allowed to stand for 15 minutes. Each test sample was judged to be "+" when the color development was able to be visually confirmed at the application positions of both the anti-mouse IgG antibody and the anti-*Mycoplasma pneumoniae* antibody. Each test sample was judged to be "-" when the color development was able to be visually confirmed only at the application position of the anti-mouse IgG antibody and the color development was not able to be visually confirmed at the application position of the anti-*Mycoplasma pneumoniae* antibody. Each test sample was judged invalid when the color development was not able to be visually confirmed at the application position of the anti-mouse IgG antibody.

The results of each test strip are shown in Table 3.

**[Table 3]**

| | ×32 | ×64 | ×128 | ×256 | ×512 | ×1024 | ×2048 |
|---|---|---|---|---|---|---|---|
| P1 | + | - | - | - | - | - | - |
| P30 | + | + | + | - | - | - | - |
| P1P30A | + | + | + | + | + | - | - |
| P1P30B | + | + | + | + | + | + | - |

As shown in Table 3, the immunoassay instrument using P1-30Bab which is a monoclonal antibody of the present invention showed higher sensitivity than the immunoassay instruments using other antibodies.

## Claims

1. An immunoassay method of *Mycoplasma pneumoniae,* which utilizes an antigen-antibody reaction between a monoclonal antibody that specifically reacts with P1 protein and P30 protein of *Mycoplasma pneumoniae* and that specifically reacts with a peptide containing an amino acid sequence composed of PPQPG, or an antigen-binding fragment thereof, and P1 protein and P30 protein derived from *Mycoplasma pneumoniae.*

2. The immunoassay method of claim 1, wherein said amino acid sequence of said peptide is PPQPGFPPKR or GMPPQPGFPPKR.

3. The method of claim 1, wherein said immunoassay method is a sandwich method in which said monoclonal antibody or an antigen-binding fragment thereof is used in at least one of a label and a solid phase.

4. The method of claim 2, wherein said immunoassay method is an immunochromatography method.

5. The method of any one of claims 1 to 4, wherein said two or more kinds of antigens recognized by said monoclonal antibody form a composite.

6. An immunoassay instrument for carrying out the method of claim 1, wherein said monoclonal antibody or an antigen-binding fragment thereof is used in at least one of a label and a solid phase.

7. The immunoassay instrument of claim 6, wherein said immunoassay instrument is an immunochromatographic strip.

8. The immunoassay instrument of claim 6 or 7, wherein said test subject is *Mycoplasma pneumoniae.*

9. A monoclonal antibody which specifically reacts with P1 protein and P30 protein of *Mycoplasma pneumoniae* and which specifically reacts with a peptide containing an amino acid sequence composed of PPQPG.
